# EUROPEAN PATENT SPECIFICATION

(11) **EP 2 150 568 B1**
(45) Date of publication and mention of the grant of the patent: **23.03.2016**
(21) Application number: 08765990.0
(22) Date of filing: 29.05.2008
(51) Int. Cl.: C08G 65/48, C08G 65/00

(54) **CHAIN-END FUNCTIONALIZED METHOXY POLY(ETHYLENE GLYCOL)AND METAL NANO-PARTICLES USING THE SAME**
AM KETTENENDE FUNKTIONALISIERTES METHOXY-POLY(ETHYLENGLYKOL) UND DAMIT HERGESTELLTES METALLNANOPARTIKEL
MÉTHOXY POLY(ÉTHYLÈNE GLYCOL) À EXTRÉMITÉ DE CHAÎNE FONCTIONNALISÉE ET NANOPARTICULES MÉTALLIQUES UTILISANT CELUI-CI

(30) Priority: 29.05.2007 KR 20070051997
(43) Date of publication of application: 10.02.2010
(73) Proprietor: Youl Chon Chemical Co. Ltd., Seoul 156-709 (KR)
(72) Inventor: SHIN, Dong-Youn, Seoul 140-200 (KR); KIM, Ji Hee, Bucheon-si Gyeonggi-do 421-815 (KR)
(74) Representative: Thurston, Joanna
(86) International application number: PCT/KR2008/003028
(87) International publication number: WO 2008/147128

(56) References cited:
- EP-A1- 0 824 126
- EP-A1- 0 864 362
- EP-A1- 0 985 697
- WO-A1-98/35248
- WO-A1-2007/024066
- WO-A2-02/059179
- WO-A2-2005/010075
- KR-A- 20040 014 687
- US-A- 4 252 677
- US-A- 5 672 662
- HERMAN S ET AL: "POLY(ETHYLENE GLYCOL) WITH REACTIVE ENDGROUPS: I. MODIFICATION OF PROTEINS", JOURNAL OF BIOACTIVE AND COMPATIBLE POLYMERS, LANCASTER, PA, US, vol. 10, no. 2, 1 April 1995 (1995-04-01), page 145, XP009032001,
- LI JING ET AL: "Synthesis of polyethylene glycol (PEG) derivatives and PEGylated-peptide biopolymer conjugates", BIOMACROMOLECULES, AMERICAN CHEMICAL SOCIETY; US, vol. 4, no. 4, 17 May 2003 (2003-05-17), pages 1055-1067, XP002328259, ISSN: 1525-7797, DOI: 10.1021/BM034069L

## Description

### [Technical Field]

Herein it is described a chain-end functionalized methoxy poly(ethylene glycol) (mPEG) and a process of preparing the same. Also it is described a living methoxy poly(ethylene glycol) for preparing the functionalized methoxy poly(ethylene glycol). Further, it is described a nano-particles of transition metal or metal salt encapsulated in the micelle structure formed by the chain-end functionalized methoxy poly(ethylene glycol). In addition, it is described a method for preparing the nano-particles of transition metal or metal salt.

### [Background Art]

Various methods for functionalizing the chain-end of PEO, which is useful for capsulating water-insoluble drugs, and their applications have been studied for a long time (J.M. Harris et al, Nature Reviews Drug Discovery, 2003, Vol. 2, pages 214- 221; Zalipsky et al, Bioconjugate Chemistry, 1995, Vol. 6, pages 150-165). In this respect, the processes for preparing polyethylene oxide or poly(ethylene glycol) by living anionic polymerization are well described in various literatures (e.g., S. Slomkowski et al, "Anionic Ring-opening Polymerization", in Ring-Opening Polymerization: Mechanism, Catalysis, Structure, Utility, Edited by D. J. Brunelle, 1993, Chap. 3, pages 87-128; Quirk et al, "Macromonomers and Macromonomers", in Ring- Opening Polymerization: Mechanism, Catalysis, Structure, Utility, Edited by D. J. Brunelle, 1993, Vol. 9, pages 263-293).

Further, the process for preparing block copolymers consisting of PEO and other polymers are also disclosed in various literatures (e.g., Jankova et al, Macromolecules, 1998, Vol. 31, pages 538-541; Topp et al, Macromolecules, 1997, Vol. 30, pages 8518-8520).

On the other hand, polymeric electrolytes prepared by polymerizing vinylic monomers having a carboxylic acid, sulfonic acid, amine or ammonium group have been used as pH-responsive hydrogels (R.S. Harland et al, "Polyelectrolyte Gels, Properties, Preparation, and Applications," ACS Symp. Series # 480, Am. Chem. Soc, Washington, D.C., 1992, Chap. 17, page 285).

### [Disclosure]

The subject-matter of the present invention is defined in claims 1-6 as attached. Embodiments described herein which are not covered by the claims merely serve to illustrate the technical context of the present invention.

Herein it is described a living mPEG of the following formula wherein the end group is replaced with an alkali metal cation: 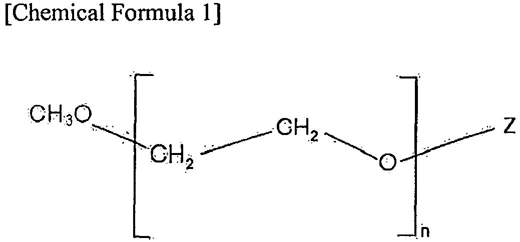 wherein, Z is selected from the group consisting of Lithium, Sodium, Potassium, Cesium and Rubidium.

Herein it is described a chain-end functionalized mPEG selected from the group consisting of compounds of the following formulas 2 to 5: 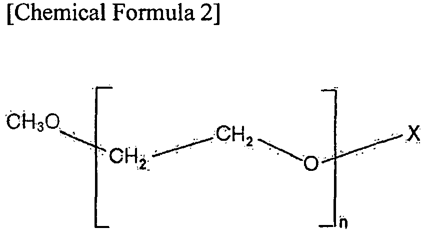 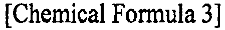 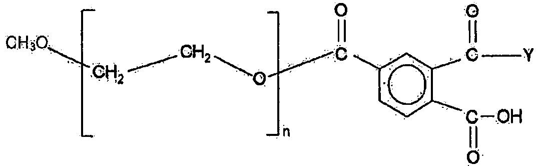 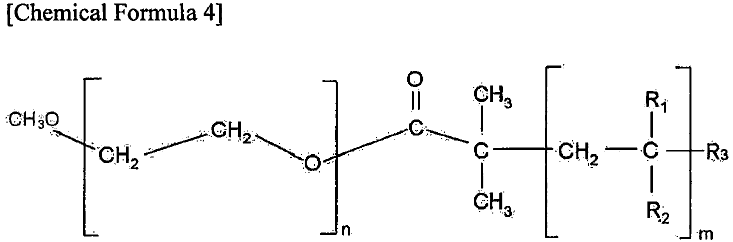 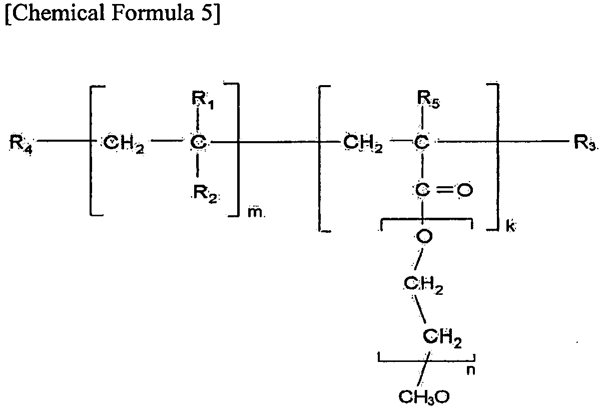 wherein,
R₁ and R₅ are each independently hydrogen or methyl,
R₂ is an amide such as N-isopropylacrylamide; a sulfonamide such as sulfabenzene, sulfisoxazole, sulfacetamide, sulfamethizole, sulfadimethoxine, sulfadiazine, sulfamethoxy pyridazine, sulfamethazine, sulfisoimidine and sulfapyridine; a vitamin such as folic acid; or amide group or sulfonamide group-containing a drug such as indisulam, doxorubicin, paclitaxel vancomycin and amprenavir,
R₃ is hydrogen, isobutylacrylonitryl, phenyl or halogen,
R₄ is phenyl or isobutylacrylonitryl,
X is hydrogen, hydroxyl (-OH), sulfonic acid (-SO₃H), thiol (-SH), carboxy (-COOH), sulfonamide (-SO₂NH-), 2-bromoisobutyryl, 2- bromopropionyl, methacrylate or anhydride,
Y is sulfonamide group such as sulfabenzene, sulfisoxazole, sulfacetamide, sulfamethizole, sulfadimethoxine, sulfadiazine, sulfamethoxy pyridazine, sulfamethazine, sulfisoimidine and sulfapyridine; a vitamin such as folic acid; or an amide or sulfonamide group-containing drug such as indisulam, doxorubicin, paclitaxel, vancomycin and amprenavir;
n is an integer in the range of 10 to 500,
k is an integer in the range of 1 to 10, and
m is an integer in the range of 5 to 50.

Herein it is described a method for preparing a chain-end functionalized methoxy poly(ethylene glycol) (mPEG) having the structure of the chemical formula 2, comprising (a-2) conducing reaction of a mPEG having a number-average molecular weight (Mn) of 500 to 20,000 g/mol with an alkyl alkali metal to provide a living mPEG having end group substituted with an alkali metal cation; and (b-2) reacting the living mPEG obtained in step (a-2) with a functionalization material under vacuum to provide a chain-end functionalized mPEG.

Herein it is described a method for preparing a chain-end functionalized methoxy poly(ethylene glycol) (mPEG) having the structure of the chemical formula 3, comprising (a-3) reacting a mPEG having a number-average molecular weight (Mn) of 500 to 20,000 g/mol with an alkyl alkali metal to provide a living mPEG having end group substituted with the an alkali metal cation; (b-3) reacting the living mPEG obtained in step (a-3) with trimellitic anhydride chloride under vacuum, or argon gas stream or nitrogen gas stream; and (c-3) reacting ω-anhydride mPEG with functionalization material under vacuum, or argon gas stream or nitrogen gas stream.

Herein it is described a method for preparing a chain-end functionalized methoxy poly(ethylene glycol) (mPEG) having the structure of the following chemical formula 4, comprising (a-4) conducting reaction of a mPEG having a number-average molecular weight (Mn) of 500 to 20,000 g/mol with an alkyl alkali metal to provide a living mPEG having end group substituted with an alkali metal cation; (b-4) funtionalizing a chain end of the living mPEG obtained in step (a-4) with 2-bromoisobutyryl or 2-bromopropionyl under vacuum; and (c-4) carrying out atom transfer radical polymerization using the mPEG having brome group in a chain end as an initiator with sulfonamide methacrylate monomers or N-isopropylacrylamide monomers to prepare a block copolymer.

Herein it is described a method for preparing a chain-end functionalized methoxy poly(ethylene glycol) (mPEG) having the structure of the following chemical formula 5, comprising (a-5) reacting a mPEG having a number-average molecular weight (Mn) of 500 to 20,000 g/mol with an alkyl alkali metal to provide a living mPEG having end group substituted with an alkali metal cation; (b-5) reacting the living mPEG obtained in step (a-5) with methacryloyl chloride to provide mPEG having a chain end funtionalized with methacrylate; and (c-5) carrying out radical polymerization using the mPEG having a chain end funtionalized with methacrylate having brome group in a chain end as a macromonomer with sulfonamide methacrylate monomers or N-isopropylacrylamide monomers to prepare a graft copolymer.

Herein it is described a polymer-drug complex, consisting of a polymer and a drug combined with the polymer, and being the chain-end functionalized mPEG mentioned above wherein R₂ and Y is selected from the group consisting of a vitamin such as folic acid; and amide group or sulfonamide group-containing a drug such as indisulam, doxorubicin, paclitaxel vancomycin and amprenavir.

Herein it is described a nano-sized transition metal or metal salt particle, wherein transition metals or metal salts thereof are encapsulated in a micelle structure formed by the chain end-funtionalized mPEG or the polymer-drug complex.

Herein it is described a method for preparing the nano-sized transition metal or metal salt particle, comprising dissolving the chain end functionalized mPEG and a transition metal-containing compound such as metal salts or hydrates in a solvent and conducting reaction between them under the presence of a reducing agent.

### [Advantageous Effects]

The present invention is capable of simply preparing polymeric medicine such as a mPEG-based polymer and graft or block copolymer exhibiting pH- or thermo-responsive property, wherein various functional materials (e.g., vitamin, anti-cancer agent, sulfonamide material, etc.) are attached to the chain-end of mPEG having a specific molecular weight. Further, the present invention is capable of simply preparing nano-sized transition metal or metal salt particles having a size from 1 to 500 nm, preferably 1 to 100 nm, using said various chain-end functionalized mPEG-based polymers. Thus, the present invention can be advantageously used in development of new materials which are useful matrices for drug delivering system, e.g., a contrast agent and an anti-cancer agent simultaneously.

### [Description of Drawings]

Fig. 1 is a NMR data of the living mPEG prepared according to the Example 1.
Fig. 2 is a GPC data of the living mPEG prepared according to the Example 1.
Fig. 3 is a NMR data of the mPEG macroinitiator prepared according to the Example 2.
Fig. 4 is a GPC data of the mPEG macroinitiator prepared according to the Example 2.
Fig. 5 is a NMR data of the mPEG macromonomer prepared according to the Example 3.
Fig. 6 is a GPC data of the mPEG macromonomer prepared according to the Example 3.
Fig. 7 is a NMR data of the ω-sulfonated mPEG prepared according to the Example 4.
Fig. 8 is a GPC data of the ω-sulfonated mPEG prepared according to the Example 4.
Fig. 9 is a NMR data of the ω-thiolated mPEG prepared according to the Example 5.
Fig. 10 is a GPC data of the ω-thiolated mPEG prepared according to the Example 5.
Fig. 11 is a NMR data of the ω-anhydride mPEG prepared according to the Example 6.
Fig. 12 is a GPC data of the ω-anhydride mPEG prepared according to the Example 6.
Fig. 13 is a NMR data of the mPEG-doxorubicin prepared according to the Example 7.
Fig. 14 is a GPC data of the mPEG-doxorubicin prepared according to the Example 7.
Fig. 15 is a NMR data of the mPEG-sulfamethazine prepared according to the Example 8.
Fig. 16 is a GPC data of the mPEG-sulfamethazine prepared according to the Example 8.
Fig. 17 is a NMR data of the mPEG-TMA-folic acid prepared according to the Example 9.
Fig. 18 is a GPC data of the mPEG-TMA-folic acid prepared according to the Example 9.
Fig. 19 is a NMR data of the mPEG-vancomycin prepared according to the Example 10.

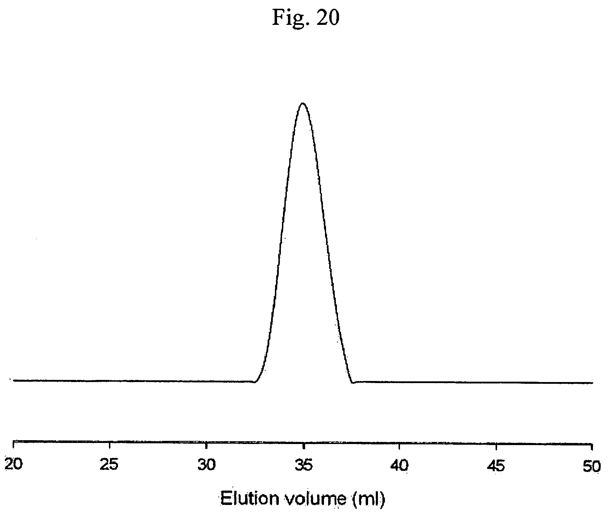

Fig. 20 is a GPC data of the mPEG-vancomycin prepared according to the Example 10.
Fig. 21 is a NMR data of the mPEG-g-NiPAM copolymer prepared according to the Example 11.
Fig. 22 is a GPC data of the mPEG-g-NiPAM copolymer prepared according to the Example 11.
Fig. 23 is a NMR data of the mPEG-g-MASX copolymer prepared according to the Example 12.
Fig. 24 is a GPC data of the mPEG-g-MASX copolymer prepared according to the Example 12.
Fig. 25 is a NMR data of the mPEG-b-MASX copolymer prepared according to the Example 13.
Fig. 26 is a GPC data of the mPEG-b-MASX copolymer prepared according to the Example 13.
Fig. 27 is a TEM photograph of the nano-particles of mPEG-b-MASX and iron oxide prepared according to the Example 14.
Fig. 28 is a TEM photograph of the nano-particles of ω-sulfonated mPEG and iron oxide prepared according to the Example 15.
Fig. 29 is a TEM photograph of the nano-particles of ω-thiolated mPEG and Au prepared according to the Example 16.
Fig. 30 is a TEM photograph of the nano-particles of ω-sulfonated mPEG and Au prepared according to the Example 17.
Fig. 31 is a TEM photograph of the nano-particles of mPEG-TMA-doxorubicin and iron oxide prepared according to the Example 18.
Fig. 32 is a TEM photograph of the nano-particles of mPEG-TMA-folic acid and iron oxide prepared according to the Example 19.
Fig. 33 is a TEM photograph of the nano-particles of mPEG-g-MASX and iron oxide prepared according to the Example 20.
Fig. 34 is a NMR data of mPEG-sulfadiazine prepared according to the Example 21.
Fig. 35 is a TEM photograph of the nano-particles of ω-sulfonated mPEG and cadmium sulfide prepared according to the Example 22.
Fig. 36 is a TEM photograph of the nano-particles of ω-thiolated mPEG and cadmium sulfide prepared according to the Example 23.
Fig. 37 is a TEM photograph of the nano-particles of mPEG vancomycin and silver prepared according to the Example 24.
Fig. 38 is a TEM photograph of the nano-particles of mPEG-TMA-folic acid and silver prepared according to the Example 25.
Fig. 39 is a TEM photograph of the nano-particles of ω-thiolated mPEG and silver prepared according to the Example 26.

### [Best Mode]

Preferred examples will now be described in detail.

Any one compound of the above chemical formulas 2 to 5 may be prepared so that the compound includes sulfonic acid group (-SO₃H), thiol group (-SH), carboxyl group (-COOH) or sulfonamide group (-SO₂NH-). For instances, sulfonic acid group (-SO₃H), thiol group (-SH) and carboxyl group (-COOH) may be introduced in a chain-end by adding 1,3-propane sultone, propylene sulfide monomers or carbon dioxide, respectively, to the living mPEG obtained in the above (a-2), (a-3), (a-4) or (a-5) (hereinafter, all of these are referred to as simply "(a)") steps.

In addition, polymeric medicine wherein a drug is introduced to a chain-end (Y group, R₂) of mPEG may be prepared by having the compound of the chemical formula 3 react with vitamin such as folic acid; or amide (NH₂) or sulfonamide (-SO₂NH- group) - based drug such as amprenavir, doxorubicin, paclitaxel and vancomycin.

The "n" of the above chemical formulas is preferably an integer of 10 to 500. This is because if the molecular weight is not limited to the range, the reactivity is remarkably reduced and the yield of the reaction becomes bad. Further, "k" is preferably an integer of 1 to 10 and "m" is preferably an integer of 5 to 50. This is because the macroinitiator of the chemical formula 4 and the macro monomer of the chemical formula 5 may trigger structurally steric hindrance.

In a method for preparing a chain-end functionalized mPEG, the mPEG, the starting material of (a) step has preferably the molecular weight of 500 to 20,000 g/mol. When the molecular weight is not limited to the range, the reactivity may be remarkably reduced due to steric hindrance, etc. and result in a low reaction yield.

In (a) step, a living mPEG having a chain-end substituted with an alkali metal cation may be prepared by having mPEG react with an alkyl alkali metal. The alkyl alkali metal may be one or more selected from the group consisting of alkyl lithium, diisopropyl amino lithium, and an alkyl alkali metal having Sodium, Potassium, Cesium or Rubidium substituted for the above-mentioned Lithium. Most preferable alkyl alkali metal is butyl lithium.

In the method for preparing a chain-end functionalized mPEG, (b-2), (b-3), (b-4) and (b-5) (hereinafter, all of these are referred to simply as "(b)") steps may be accomplished by having the living mPEG obtained in step a) react with sultone (e.g., 1,3-propane sultone and 1,4-butane sultone), ethylene sulfide, propylene sulfide, trimellitic anhydride chloride, methacryloyl chloride, 2-bromoisobutyryl bromide, 2-bromopropionyl bromide or 2- bromopropionyl chloride, etc., under vacuum, or argon gas stream or nitrogen gas stream.

The solvent used in step b) may be benzene/DMSO or benzene/methanol/DMSO. Also, the functionalization step b) may be conducted at a temperature of 20 to 80 °C for 6 to 48 hours.

Further, in step b), diverse functional groups can be introduced quantitatively into the chain-end of mPEG and the chain-end functionalized mPEGs of the chemical formulas 2 to 5 may be prepared accordingly. The functional groups include, but not limited to: hydrogen, hydroxyl group (-OH), sulfonic acid group (-SO₃H), thiol group (-SH), carboxyl group (-COOH), sulfonamide group (^-SO₂NH-); vitamin group such as folic acid; and amine- or sulfonamide- based drug group such as doxorubicin, paclitaxel, vancomycin and amprenavir.

In addition, anhydride-containing mPEG may be prepared by having the living mPEG obtained in step a) react with trimellitic anhydride chloride. The resulting anhydride-containing mPEG reacts with vitamin group such as folic acid; or an amine- or sulfonamide-based drug such as doxorubicin, paclitaxel, vancomycin and amprenavir in a solvent such as water or methanol to obtain a polymeric medicine of the chemical formula 3, wherein a drug is introduced into a chain-end of mPEG.

(c-3), (c-4) and (c-5) (hereinafter, all of these are referred to simply as "(c)") is steps for preparing a graft or block copolymer. Specifically, the block copolymer of formula 4 may be obtained by having the macroinitiator (e.g., the compound of formula 2 wherein X is 2-bromoisobutyryl or 2- bromopropionyl) react (atom transfer radical polymerization) with N-isopropyl acrylamide (NiPAM) or sulfonamide methacrylamide monomer such as sulfadiazine in a solvent in the presence of a catalyst system.

Further, the graft copolymer of formula 5 exhibiting thermo- or pH-responsive properties may be obtained by having the macromonomer (e.g., the compound of formula 2 wherein X is methacrylate) react (radical polymerization) with NiPAM, or sulfonamide methacrylate monomer such as sulfadiazine in a solvent in the presence of an initiator (e.g., benzoyl peroxide (BPO) or azobisisobutyronitril (AIBN)).

The solvent suitable for use in step c) may be water or cyclohexane, or a mixture of a non-polar sovent such as benzene or toluene and a polar solvent such as tetrahydrofuran (THF) and dimethylsulfoxide (DMSO). The mixing volume ratio of the non-polar / polar solvent may be 90/10 to 70/30. The initiator may be benzoyl peroxide (BPO), 2,2'-azobisisobutyronitrile (AIBN). A copper-based atom transfer radical polymerization (ATRP) catalyst etc. may be used. Also, the radical polymerization step c) may be conducted preferably at a temperature of 20 to 80 °C.

As described above various functional groups including a drug can be effectively introduced into mPEG having a specific molecular weight. In addition, the nano-sizing of transition metal-containing compound such as metal salts or metal hydrates can be easily achieved using the chain-end functionalized mPEG. The nano-sized metal or metal salt particles are obtained in a form of polymer-encapsulated particles wherein the polymer is a water-soluble mPEG-based material. As such, they can be readily soluble in an aqueous medium as well as in an organic solvent.

Here, the term "transition metal-containing compound" means all of the compounds containing a transition metal. The transition metal-containing compound includes, but not limited to, transition metals or metal hydrates. The transition metal-containing compound may preferably be one or more selected from the group consisting of FeCl₃, FeCl₂, HAuCl₄, Cd(OAc)₂·XH₂O and AgNO₃.

A transition metal such as gold (Au), silver (Ag), Platinum (Pt), Palladium (Pd), cadmium sulfide (CdS), iron oxide (γ-Fe₂O₃ or Fe₃O₄), PbS, etc. or a salt thereof may be stabilized in the form of a nano-cluster. The nano-cluster has preferably a size of 1 to 500 nm, more preferably 1 to 100nm.

The nano-particles of transition metals or salts thereof include, but not limited to Au, Ag, Pt(II), Pd(II), CdS, PbS, TiO₂, γ-Fe₂O₃ and Fe₃O₄ particles.

In the method for preparing nano-particles of transition metals and salts thereof, the concentration of the transition metal-containing compound solution, which is a starting material, is preferably 0.01 to 1g/ml. The reaction temperature is preferably 5 to 70 °C and may be changed according to the desired particle size or the reaction rate. The reducing agent includes ammonium hydroxide (NH₄OH), hydrazine monohydrate (N₂H₂), NaBH₄, H₂O₂, H₂S, Na₂S etc. The molar ratio of the polymer to the metal or the metal salt is preferably from 100: 1 to 1:1. When the amount of the polymer is too large, the content of the nano-particles is too low. When the amount of the polymer is too small, the metal nano-particles are not stabilized, and non-uniformed particles and much precipitates are formed. The above method enables the preparation of the nano-cluster of metal or its salts in an aqueous solution as well as in an organic solvent.

### [Examples]

Eamples falling under claims 1-6 are intended to further illustrate the present invention without limiting its scope.

### <Example 1>

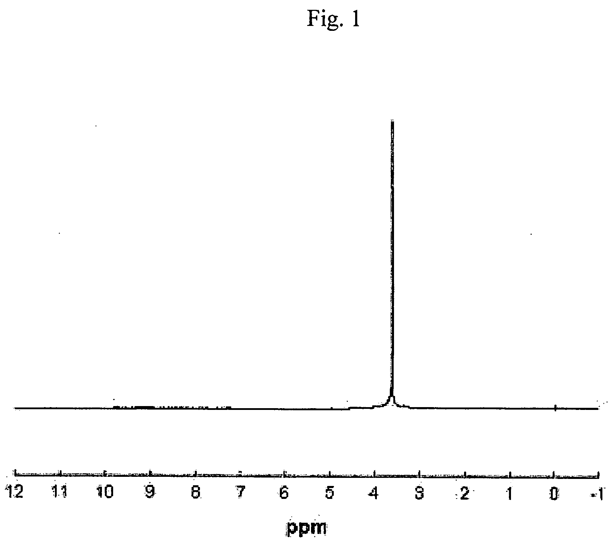

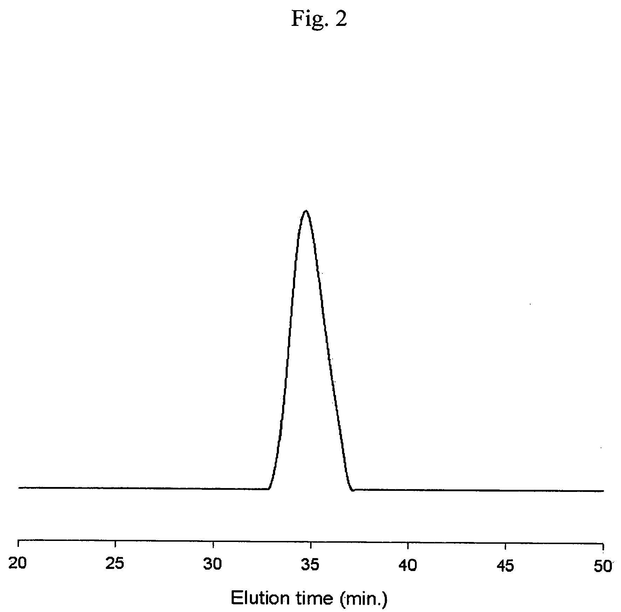

0.01 mole of mPEG (Molecular weight: 5,000 g/mol, Aldrich; Poly(ethylene glycol) methyl ether) was put to a 2L-round bottom Pyrex flask and the air was evacuated therefrom by attaching to vacuum line and dried. Then, 1L of benzene was distilled and the m-PEG was dissolved in the benzene. N-butyl lithium (30ml) was slowly added to the resulting solution with a syringe under argon gas stream while cooling using an ice bath, followed by slowly warming to about 30 °C. After 48 hours, when it was confirmed that the transparent color of the solution was gradually changed to yellow color, the reaction was terminated by adding a small amount of distilled methanol to the solution and then contacting the solution with air. The solution was precipitated in diethyl ether to obtain a living mPEG wherein an end-group is substituted with Li (mPEG-Li). The obtained polymer has a number-average molecular weight was 5,000 g/mol. Fig. 1 is a NMR data of the living mPEG obtained above and Fig. 2 is a GPC data of the living mPEG obtained above.

### <Example 2>

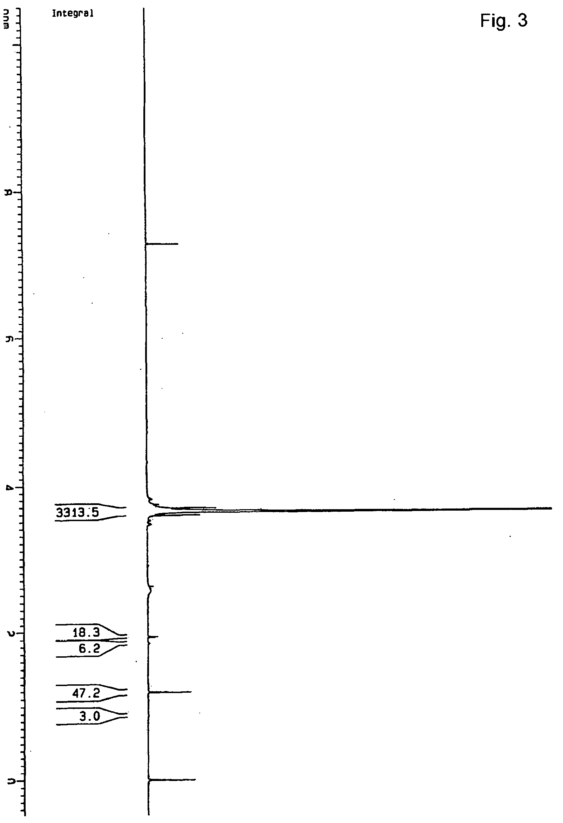

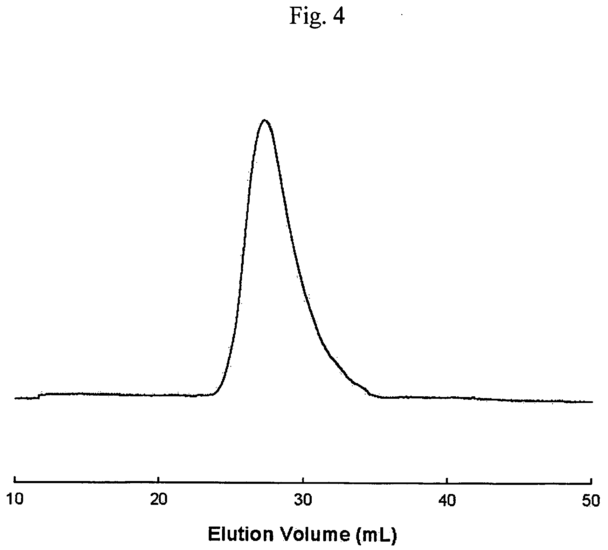

20 mmol of 2-bromoisobutyryl bromide (in 20 ml of THF) was added to 200 ml of polymeric alkoxide solution ([mPEG-Li] = 6.3 mmol) obtained in Example 1, followed by stirring for 24 hrs at room temperature. After the completion of the reaction, the solvent was removed therefrom by evaporation under reduced pressure. The resulting residue was recrystallized from ethanol to obtain powder (mPEG-based macro initiator). The number-average molecular weight of the obtained polymer was 5,300 g/mol based on a GPC analysis. The yield of chain-end bromination was 95 mol% or more based on a ¹H-NMR analysis. Fig. 3 is a NMR data of the mPEG-based macro initiator obtained above and Fig. 4 is a GPC data of mPEG based macro initiator obtained above.

### <Example 3>

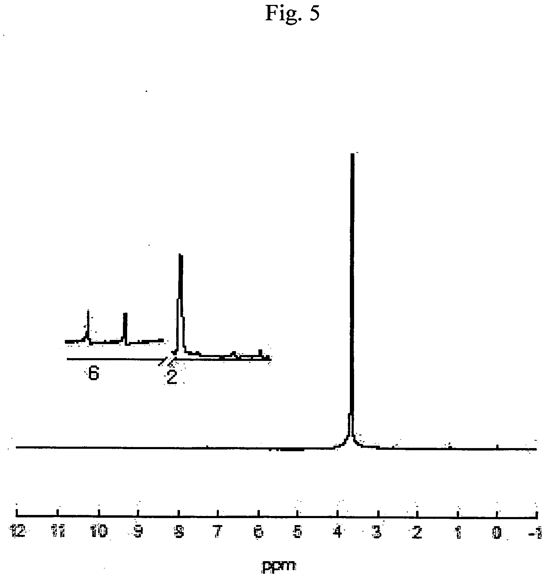

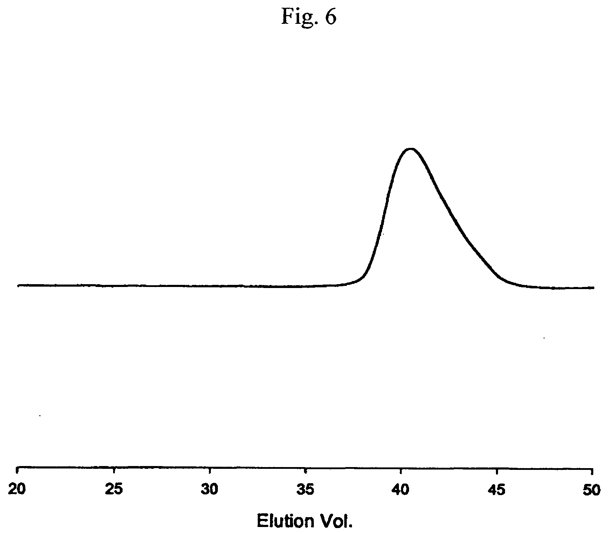

30 mmol of methacrylroyl chloride was added to 200 ml of the solution ([mPEG-Li]=6.3 mmol) of the living mPEG obtained in Example 1, followed by stirring for 24 hrs at room temperature. After the completion of the reaction, the solvent was removed therefrom by evaporation under reduced pressure. The resulting residue was re-dissolved in THF, precipitated in diethyl ether and recrystallized from ethanol to obtain the mPEG-based macromonomer. The number-average molecular weight of the obtained polymer was 5,100 g/mol. The yield of chain-end functionalization was over 95 mol% based on a ¹H-NMR analysis. Fig. 5 is a NMR data of the mPEG-based macromonomer obtained above and Fig. 6 is a GPC data of the mPEG-based macromonomer obtained above.

### <Example 4>

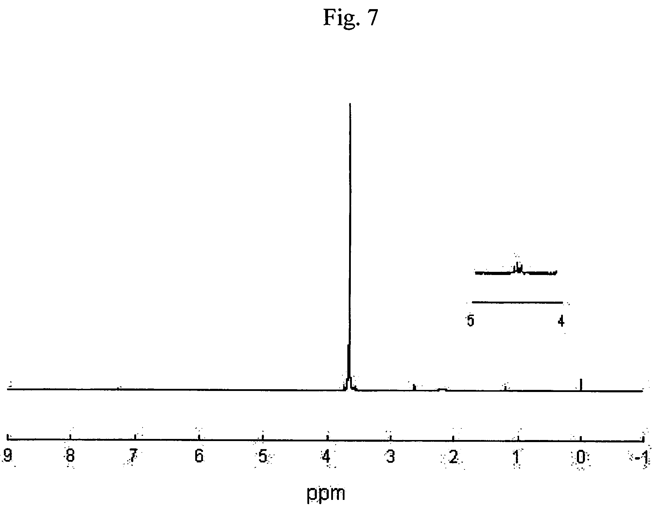

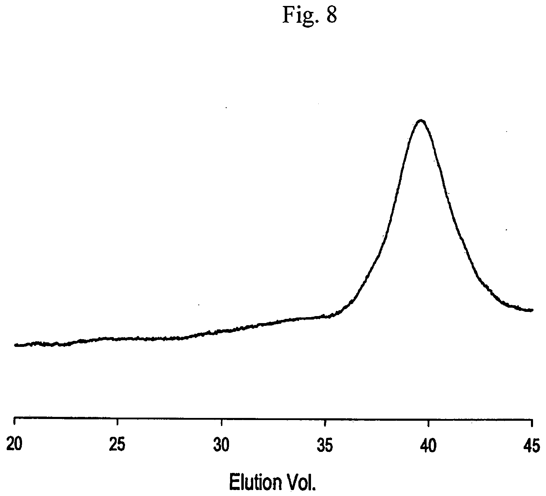

1,3-Propane sultone in THF was added ([mPEG-Li]/[sultone]=1/3, mol/mol) to 200 ml of the solution of the living mPEG (Mw=5,000 g/mol) obtained in Example 1 and the mixture was subject to reaction for 24 hrs at room temperature to obtain ω-sulfonated mPEG. Some of the solvent was removed therefrom by evaporation under reduced pressure. The resulting residue was precipitated in diethyl ether, dissolved in THF and recrystallized from ethanol to obtain powder. The number-average molecular weight of the obtained polymer was 5,100 g/mol based on a GPC analysis. The yield of chain-end functionalization was over 95 mol% based on a 1H-NMR analysis. Fig. 7 is a NMR data of the ω-sulfonated mPEG obtained above and Fig. 8 a GPC data of the ω-sulfonated mPEG obtained above.

### <Example 5>

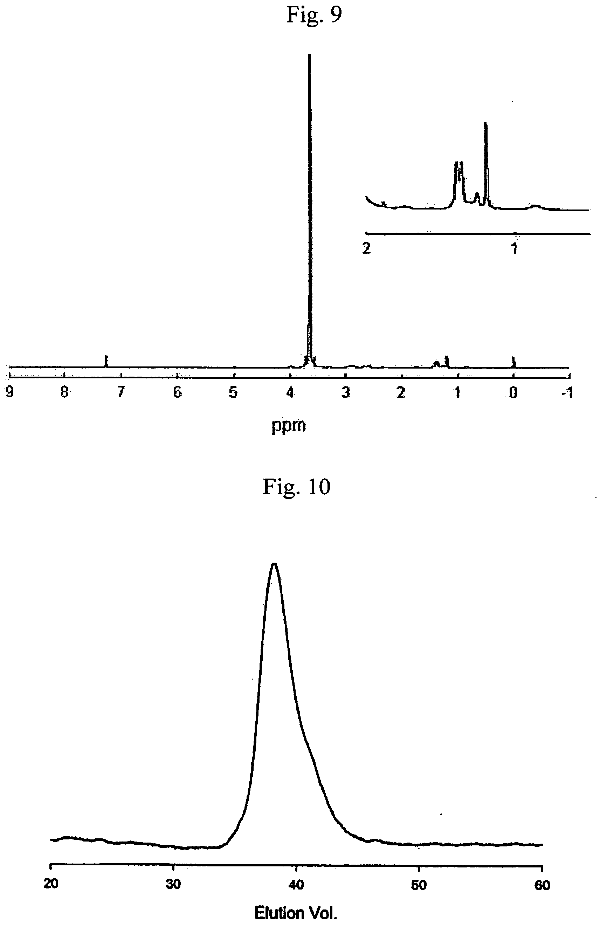

Purified propylene sulfide was added ([mPEG-Li]/[PPS] = 1/3, mol/mol) to 200 ml of the solution of the living mPEG (Mw=5,000 g/mol, 6.3 mmol) obtained in Example 1 and allowed to react for 6 hrs at room temperature under a high vacuum to introduce a thiol group into the polymer chain-end. The resulting product was recovered by precipitating it in diethyl ether, re-dissolved in THF and recrystallized from ethanol to obtain as powder. The number-average molecular weight of the obtained polymer was 5,100 g/mol based on a GPC analysis. The yield of chain-end thiolization was over 95 mol% based on a ¹H-NMR analysis. Fig. 9 is a NMR data of the ω-thiolated mPEG obtained above and Fig. 10 is a GPC data of the ω-thiolated mPEG obtained above.

### <Example 6>

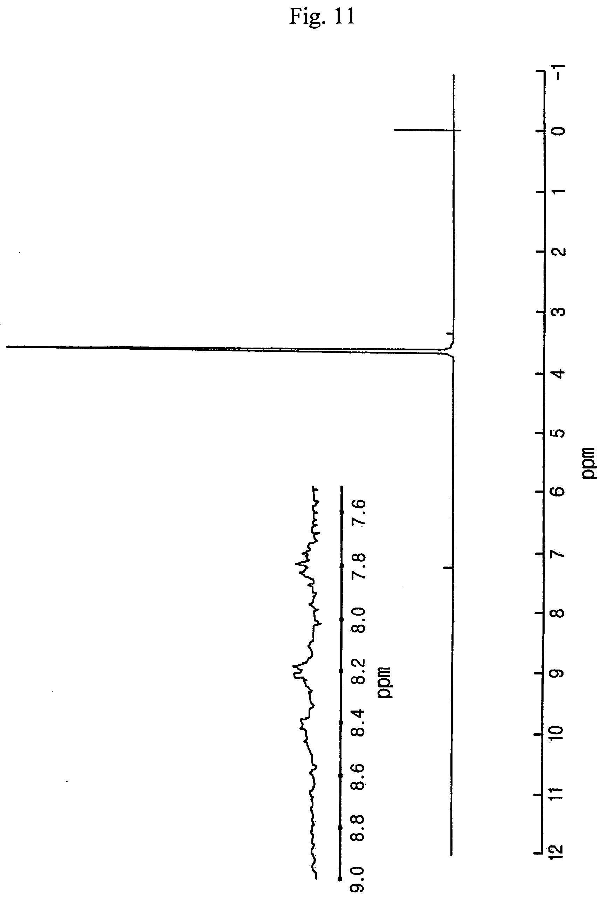

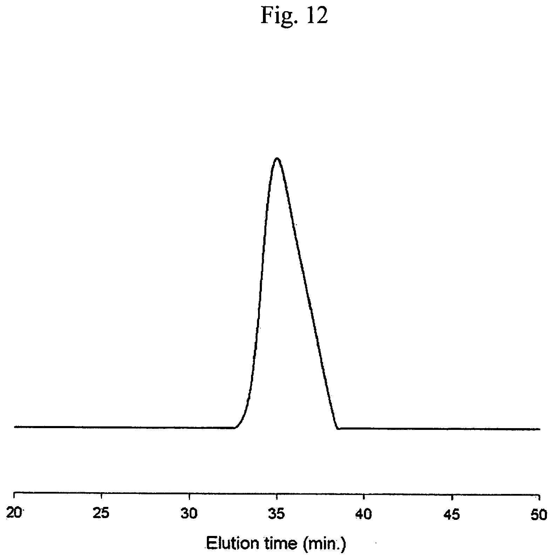

0.005 mol of trimellitic anhydride chloride (Aldrich) (98%) in 60 ml of THF was put with a syringe to a reactor containing the solution ([mPEG-Li]=0.001 mmol) of the living mPEG (Mw=5,000 g/mol) obtained in Example 1. The mixture was allowed to react for 1 hr at 5 °C and was further allowed to react for 15 hrs at 35 °C, precipitated in diethyl ether and the solvent was removed therefrom. The resulting residue was dissolved in THF and recrystallized from ethanol to obtain ώ-anhydride-mPEG. The number-average molecular weight of the obtained polymer was 5,200 g/mol. The yield of chain-end functionalization was about 85 mol% based on the concentration of the polymer solution initially used. Fig. 11 is a NMR data of the ώ-anhydride-mPEG obtained above and Fig. 12 is a GPC data of the the ώ-anhydride-mPEG obtained above.

### <Example 7>

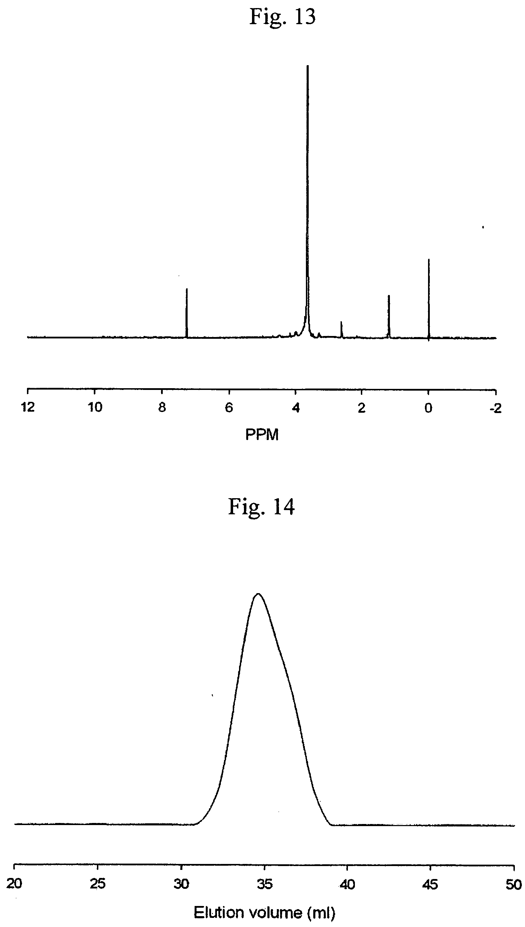

1.5 g ω-anhydride-mPEG (mPEG-TMA) (Mn = 5,200 g/mol) obtained in Example 6 and doxorubicin chloride (0.17 g)/MeOH (50 ml) were put in 100 ml reactor and allowed to react for 24 hrs under a nitrogen gas atmosphere. The resulting product was recovered by precipitating in dimethyl ether and washed several times using diethyl ether. The precipitates were dissolved in THF, and the THF-soluble and insoluble portions were separated. The THF-soluble portion contains mPEG-doxorubicin (mPEG-TMA-Dox) and the THF-insoluble portion contains unreacted doxorubicin. The THF-soluble portion was concentrated to obtain powder (mPEG-TMA-Dox) as a red solid. The obtained powder was a polymer drug of mPEG having a doxorubicin group at its chain-end. The number-average molecular weight of the obtained polymer was 5,800 g/mol. The yield of chain-end functionalization was over 95 mol% based on a ¹H-NMR analysis. Fig. 13 is a NMR data of mPEG-doxorubicin obtained above and Fig. 14 is a GPC data of mPEG-doxorubicin obtained above.

### <Example 8>

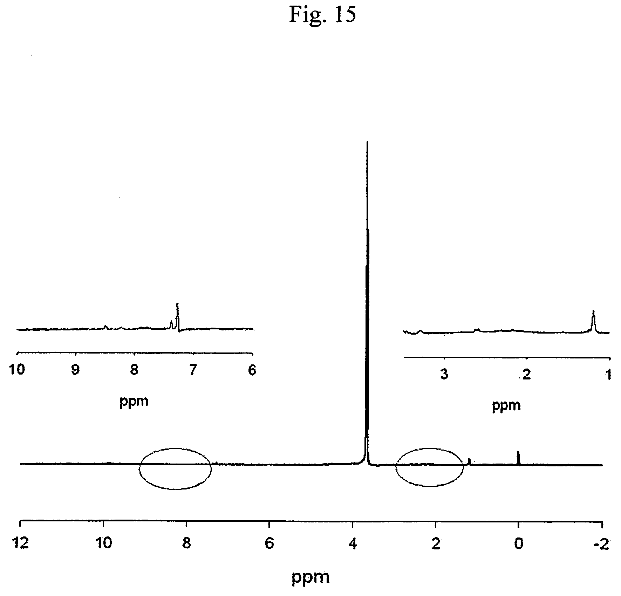

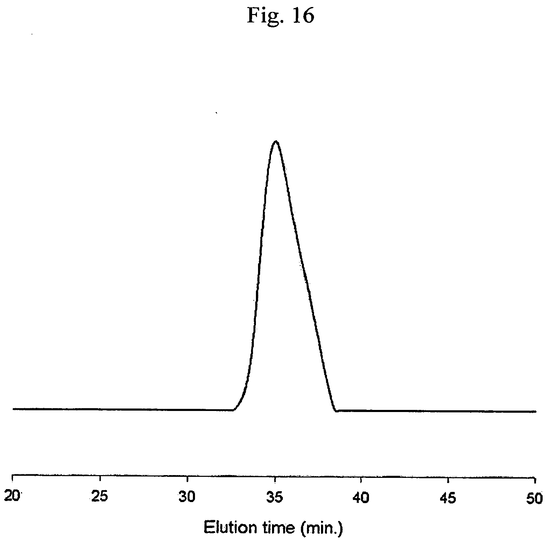

0.01 mol of mPEG-TMA (Mn = 5,200 g/mol) obtained in Example 6 and sulfamethazine (0.03 mol)/ethanol (50 ml) were put in 250 ml reactor. Then, 100 ml of ethanol was added thereto. The mixture was refluxed for 12 hrs at 70 °C while stirring. After the completion of the reaction, the resulting product was precipitated in diethyl ether at room temperature and recrystallized from ethanol to be obtained in a solid state (mPEG-sulfonamide). The number-average molecular weight of the obtained polymer was 5,400 g/mol and the reaction yield was over 95 mol% based on the amount of mPEG used. Fig. 15 is a NMR data of the mPEG-sulfamethazine obtained above and Fig. 16 is a GPC data of the mPEG-sulfamethazine obtained above.

### <Example 9>

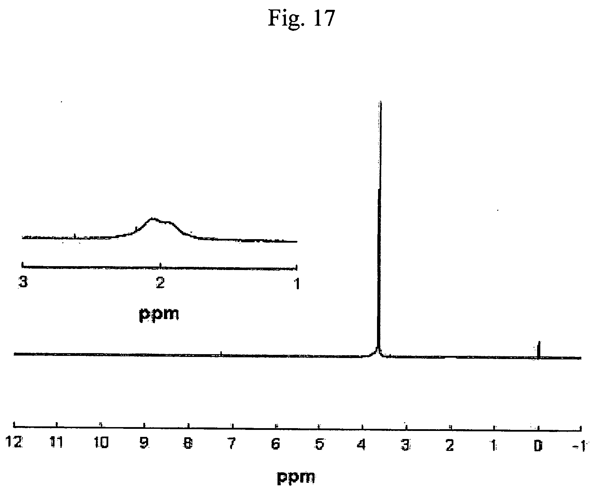

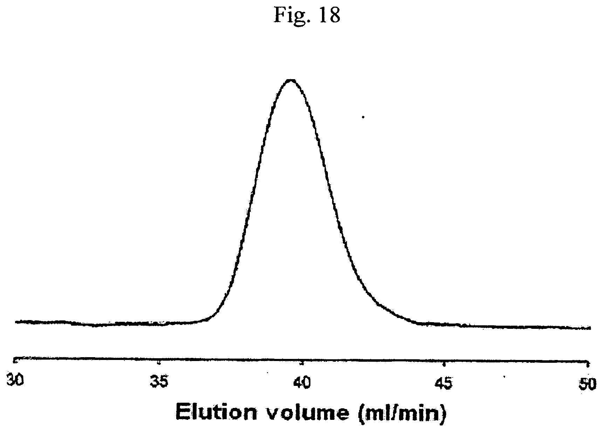

1 g of mPEG-TMA (Mn = 5,200 g/mol) obtained in Example 6 and 0.42 g of folic acid (5 eq.) were reacted in 20 ml of DMSO for 24 hrs at room temperature. The resulting product was precipitated in diethyl ether, re-dissolved in THF and recrystallized from ethanol to obtain yellow powder (mPEG-TMA-FA). The number-average molecular weight of the obtained polymer was 5,600 g/mol and the reaction yield was over 98 mol% based on the amount of mPEG used. Fig. 17 is a NMR data of the mPEG-TMA-folic acid obtained above and Fig. 18 is a GPC data of the mPEG-TMA-folic acid obtained above.

### <Example 10>

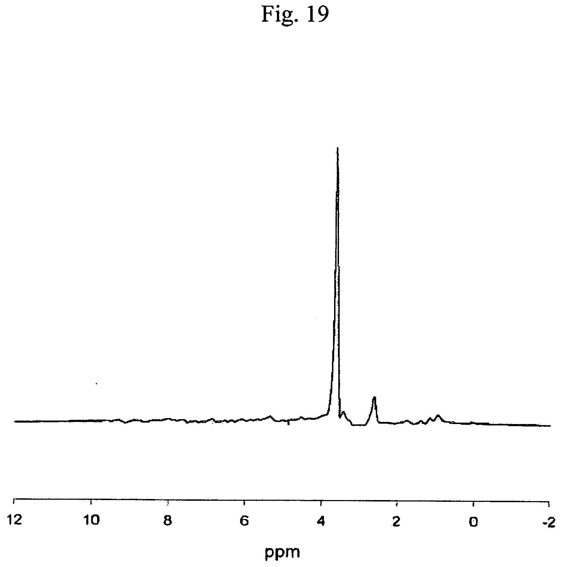

0.8 g of mPEG-TMA (Mn = 5,200 g/mol) obtained in Example 6 and 0.68 g of vancomycin (3 eq.) were reacted in 20 ml of DMSO for 80 hrs at room temperature. The resulting product was dissolved in methanol and precipitated in diethyl ether to obtain gray powder. The number-average molecular weight of the obtained polymer was 6,500 g/mol. The yield of chain-end functionalization was over 98 mol% based on a ¹H-NMR analysis. Fig. 19 is a NMR data of the mPEG-TMA-vancomycin obtained above and Fig. 18 is a GPC data of the mPEG-TMA-vancomycin obtained above.

### <Example 11>

Copolymerization of the macromonomer (1.6 mol%) obtained in Example 3 and N-isopropylacrylamide (NiPAM, 98.4 mol%) was performed as follows.

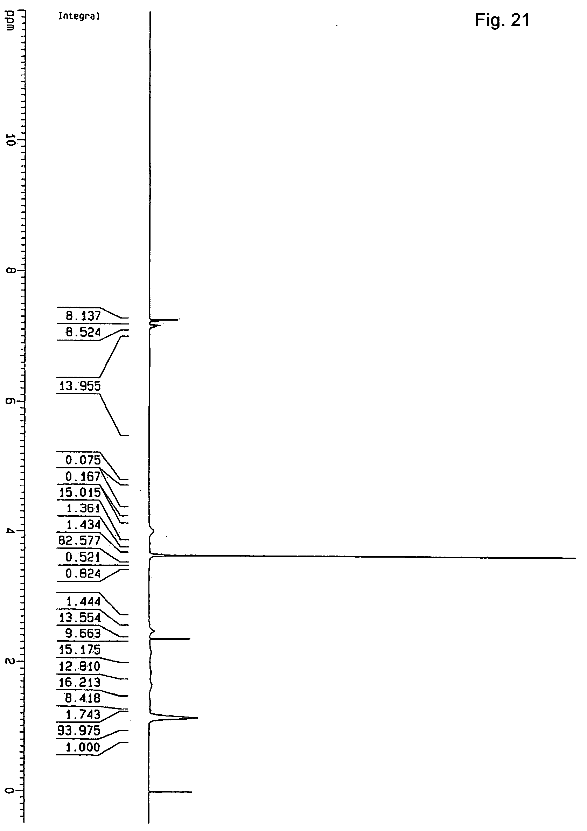

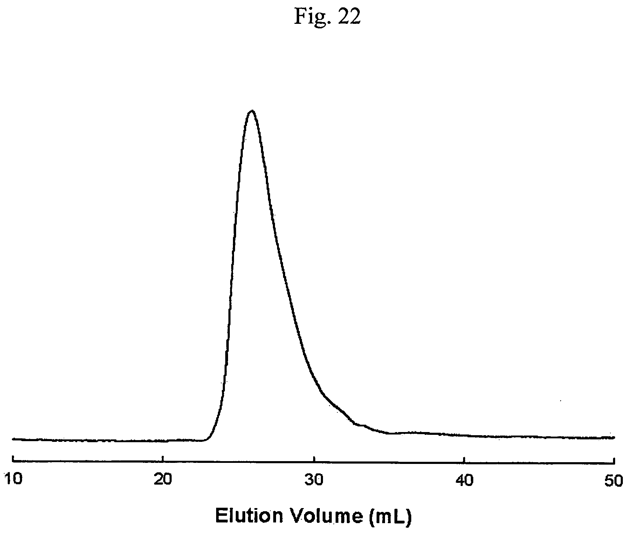

4-(Bromomethyl)benzoic acid (0.25 mmol), sodium hydroxide (0.5 mmol) and distilled water (20 ml) were put in 250 ml 3-neck flask under a nitrogen gas atmosphere. The mixture was slowly stirred for about 30 minutes. The mPEG macromonomer (2.25 g, 0.5 mmol)/distilled water (50 ml) solution was prepared in 100 ml 2-neck flask under an argon gas atmosphere. The NiPAM (3.4 g, 30 mmol)/distilled water (50 ml) solution was prepared while stirring in other 100 ml 2- neck flask under an argon gas atmosphere. The Me₆TREN (ligand, 0.25 mmol)/Cu(I)Br (0.25 mmol) mixture was added to the 250 ml flask containing the initiator. Then, the macromonomer and NiPAM solutions were added thereto simultaneously after 1 minute using a cannula and a syringe, respectively. The resulting mixture was stirred for 3 hrs at room temperature under an argon gas atmosphere. The resulting solution was precipitated in distilled water of 50 °C to obtain 4.5 g of powder. The number-average molecular weight of the obtained graft copolymer was 18,000 g/mol. Fig. 21 is a NMR data of the mPEG-g-NiPAM copolymer obtained above and Fig. 22 is a GPC data of the mPEG-g-NiPAM copolymer obtained above.

### <Example 12>

Copolymerization of the macromonomer (5 mol%) obtained in Example 3 and sulfonamide methacrylamide monomer (MASX, 95 mol%) was performed as follows.

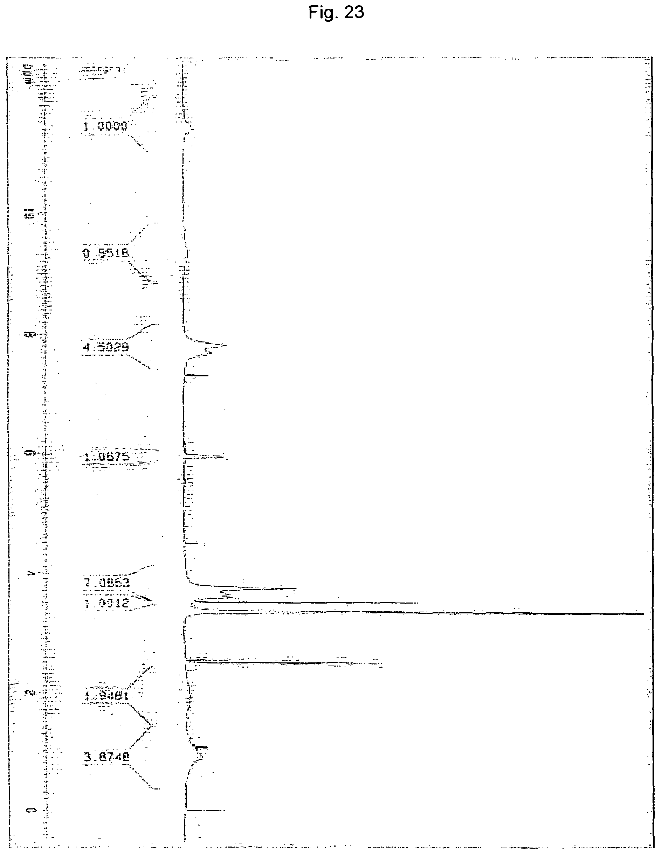

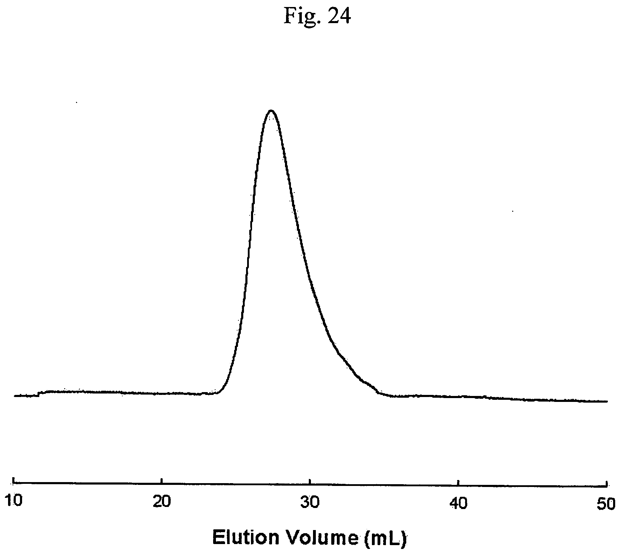

4-(Bromomethyl)benzoic acid (0.25 mmol), sodium hydroxide (0.5 mmol) and distilled water (20 ml) were put in 250 ml 3-neck flask under a nitrogen gas atmosphere. The mixture was slowly stirred for about 30 minutes. The mPEG macromonomer (2.55 g, 0.5 mmol)/distilled water (50 ml) solution was prepared in 100 ml 2-neck flask under an argon gas atmosphere. The sulfonamide methacrylamide monomer (MASX, 3.8 g, 10 mmol)/NaOH (50 mmol)/H₂O (50 ml) solution was prepared in other 100 ml 2 -neck flask under an argon gas atmosphere. The Me₆TREN (ligand, 0.25 mmol)/Cu(I)Br (0.25 mmol) mixture was added to the 250 ml flask containing the initiator. Then, the macromonomer and MASX solutions were added thereto simultaneously after 1 minute using a cannula and a syringe, respectively. The resulting mixture was stirred for 3 hrs at room temperature under an argon gas atmosphere. The reaction was terminated by adding an excess amount of HCl solution. The resulting solution was precipitated in distilled water of pH 4.5 to obtain 4.9 g of powder. The number-average molecular weight of the obtained graft copolymer was 19,000 g/mol. Fig. 23 is a NMR data of the mPEG-g-MASX copolymer obtained above and Fig. 24 is a GPC data of the mPEG-g-MASX copolymer obtained above.

### <Example 13>

Atom transfer radical polymerization was performed as follows using the mPEG having a chain-end bromide group obtained in Example 2 as an initiator.

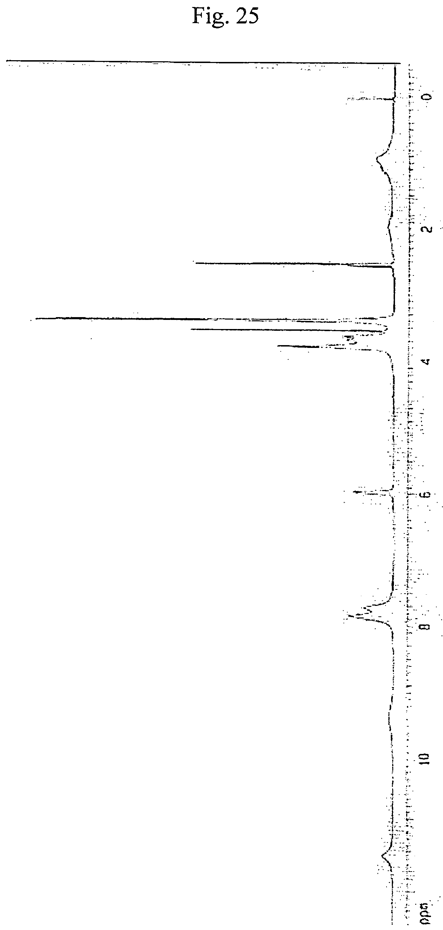

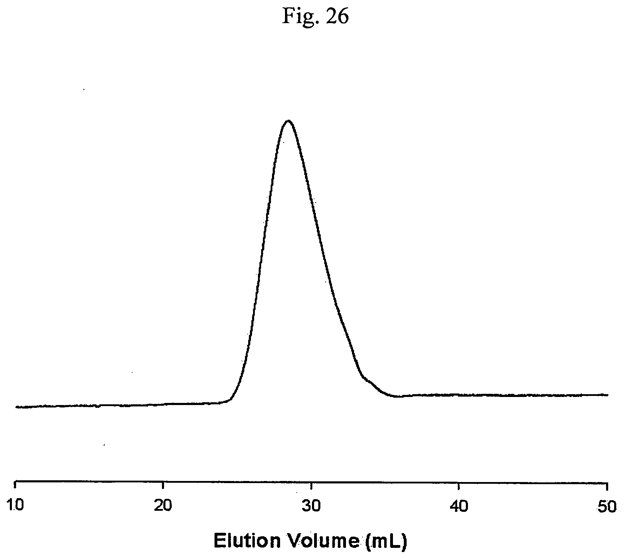

H₂O/THF (100 ml/10 ml) was put in 250 ml 3-neck flask. Then, 1.25 g of the mPEG-based macro initiator (Mn = 5,300 g/mol) was added thereto and thoroughly dissolved therewith under an argon gas atmosphere. The MASX (2.6 g, 7 mmol)/NaOH (0.301 g, 7 mmol) mixture was thoroughly dissolved in distilled water (50 ml) in 100 ml 2-neck flask. The Me₆TREN (0.25 mmol)/Cu(I)Br (0.25 mmol) mixture was added to the 250 ml flask and the mixture was stirred for about 10 minutes. To the resulting mixture, the MASX solution was added using a cannula, followed by polymerization for 2 hrs. The polymerization was terminated and the resulting solution was precipitated in an aqueous HCl solution to obtain powder. The powder were washed several times with HCl/methanol and dried in vacuum oven. The number-average molecular weight of the obtained block copolymer was 15,000 g/mol. Fig. 25 is a NMR data of the mPEG-*b*-MASX copolymer obtained above and Fig. 26 is a GPC data of the mPEG-*b*-MASX copolymer obtained above.

### <Example 14>

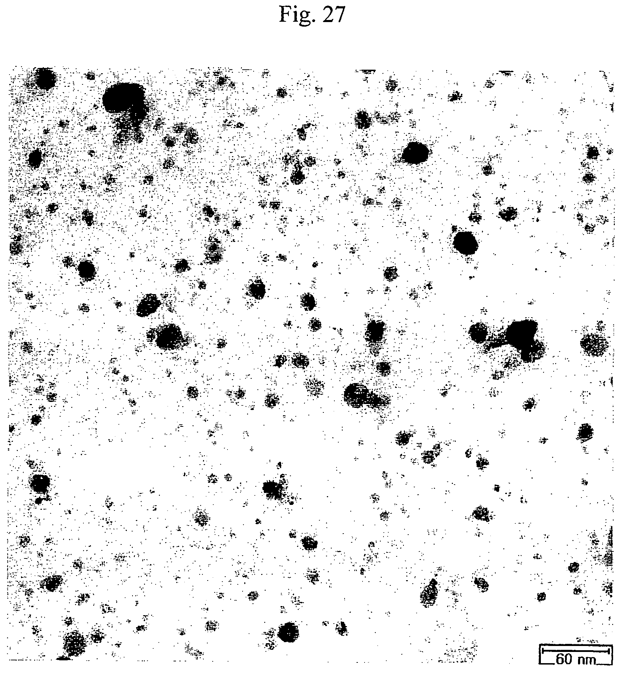

0.15 g of block copolymer (mPEG-b-poly(sulfonamide)) obtained in Example 13 was put in 20 ml vial and thoroughly dissolved with 3 ml of DMF (99%). 1 ml of FeCl₃ solution (0.146 g of FeCl₃ / 10 ml of DMF) was added thereto using a syringe. The mixture was slowly stirred for 10 minutes using a magnetic bar. The color of the solution in vial was brown. To the mixture, 1 ml of hydrazine monohydrate (N₂H₂, Wako Junyaku Co., 98%) was slowly added while stirring until the color thereof does not change any more. When the color change or bubbling no longer occurs, the resulting mixture was precipitated in an excess amount of methanol, filtered, washed and dried to obtain beige powder. The size of the powder was in the range of 2 to 20 nm based on a transmission electron microscopy (TEM) analysis. Fig. 27 is a TEM photograph of the nano-particles of mPEG-*b*-MASX iron oxide obtained above.

### <Example 15>

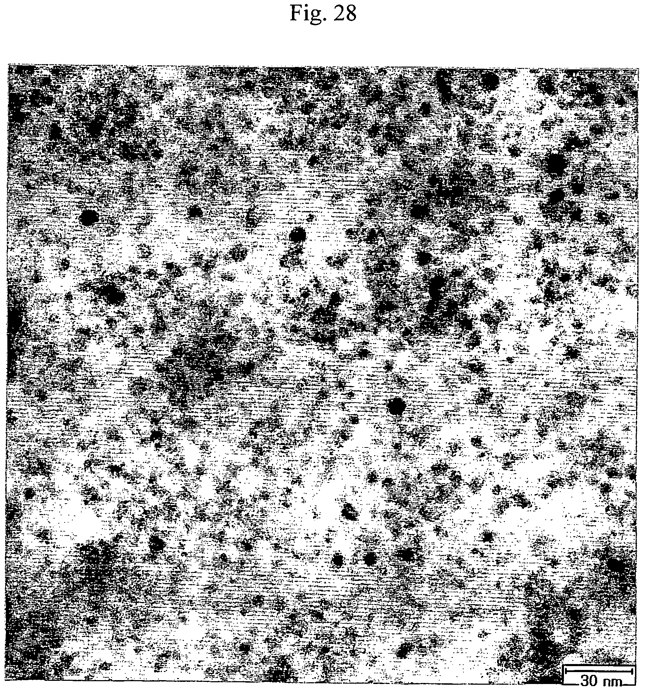

0.51 g of chain-end sulfonated mPEG obtained in Example 4 was put in 20 ml vial and thoroughly dissolved with 5 ml of DMF (99%). 2 ml of FeCl₂ solution (0.4 g FeCl₂/ 1 ml of DMF) was added thereto using a syringe. 5 ml of aqueous NaOH solution (12.5 N) was added to the mixture, warmed to 60 °C and stirred. 1.5 ml of NH₄OH was added thereto using a syringe, stirred for 6 hrs, cooled to room temperature and further stirred for 24 hrs. The brown insoluble portion was removed therefrom by filtration and the resulting solution was concentrated under a reduced pressure. The resulting residue was dissolved in methanol and precipitated in dimethyl ether to obtain yellow powder. The size of the powder was in the range of 3 to 10 nm based on a TEM analysis. Fig. 28 is a TEM photograph of the nano-particles of the ω-sulfonated mPEG-iron oxide obtained above.

### <Example 16>

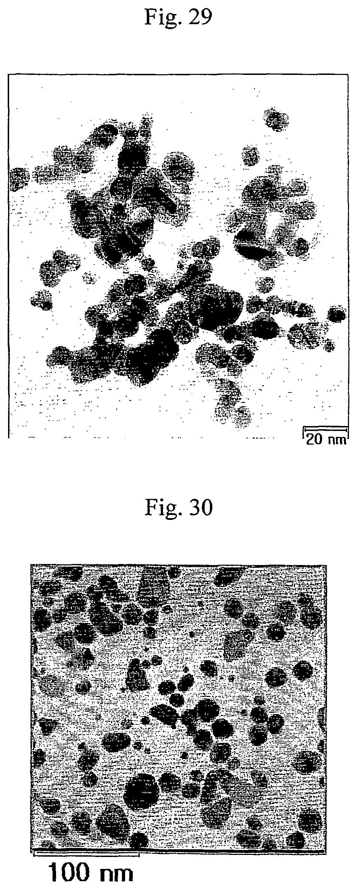

0.51 g of mPEG having a chain-end thiol group (Mn= 5,100 g/mol) obtained in Example 5 was thoroughly dissolved in 10 ml of THF. HAuCl₄ purchased from Aldrich company (2.0 x 10⁻⁴ mol) in 30 ml vial was dissolved with THF (10 ml) and NaBH₄ (1.6X 10⁻² mol) dissolved in 10 ml of THF/methanol (9/1, v/v) was added thereto using a syringe. To the mixture, the polymer solution dissolved in THF was added using a syringe, followed by stirring for 24 hrs at room temperature. Some of the solvent was removed therefrom by evaporation and the resulting residue was precipitated in dimethyl ether to obtain light purple powder. The size of the powder was in the range of 2 to 10 nm based on a TEM analysis. Fig. 29 is a TEM photograph of the nano-particles of the ω-thiolated mPEG-Au obtained above.

### <Example 17>

0.51 g of chain-end sulfonated mPEG obtained in Example 4 was put in 20 ml vial and thoroughly dissolved with 5 ml of THF (99%). HAuCl₄ purchased from Aldrich company (2.0X10⁻⁴ mol) was injected to 30 ml vial, dissolved with THF (10 ml) and NaBH4 (1.6X 10"2 mol) dissolved in 10 ml of THF/methanol (9/1, v/v) was added thereto using a syringe. To the mixture, the polymer solution dissolved in THF was added using a syringe, followed by stirring for 24 hrs at room temperature. Some of the solvent was removed therefrom by evaporation and the resulting residue was precipitated in dimethyl ether to obtain light purple powder. The size of the powder was in the range of 3 to 20 nm based on a TEM analysis. Fig. 30 is a TEM photograph of the nano-particles of the ω-sulfonated mPEG-Au obtained above.

### <Example 18>

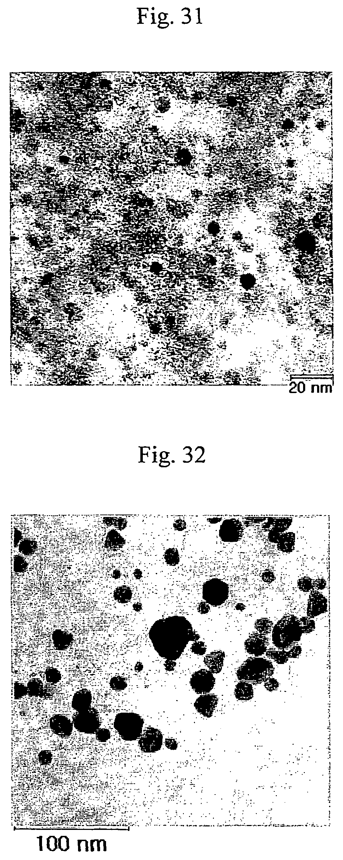

1.0 g of mPEG-TMA-Dox (Mn= 5,800 g/mol) obtained in Example 7 was put in 20 ml vial and thoroughly dissolved with 10 ml of methanol. 1 ml of FeCl₃ solution (0.48 g of FeCl₃ / 100 ml of methanol) was added thereto using a pipette. To the mixture, 1 ml of N₂H₂ was slowly added using a syringe, followed by stirring for 2 hrs. The insoluble portion was removed therefrom by filtration and the resulting solution was precipitated in diethyl ether. It was then washed several times to obtain purple powder. The powder were in the form of nanohybrid having a size ranging from 2 to 20 nm based on a TEM analysis. Fig. 31 is a TEM photograph of the nano-particles of the mPEG-TMA-doxorubicin-iron oxide obtained above.

### <Example 19>

1.5 g of mPEG-TMA-FA (Mn= 5,600 g/mol) obtained in Example 9 was dissolved in 50 ml of deoxygenated distilled water. FeCl₂/FeCl₃ (1 mol/2 mol, 0.4 g/1.0 g) was added thereto and warmed to 80 °C while stirring. To the mixture, 1.5 ml of NH₄OH solution was added, followed by stirring for 30 minutes. The resulting mixture was cooled to room temperature and further stirred for 24 hrs. From the resulting solution, the dark brown insoluble portion was removed by filtration and then water was removed. The resulting residue was dissolved in methanol and precipitated in dimethyl ether to obtain yellow powder. The size of the powder was in the range of 2 to 10 nm based on a TEM analysis. Fig. 32 is a TEM photograph of the nano-particles of the mPEG-TMA-folic acid-iron oxide obtained above.

### <Example 20>

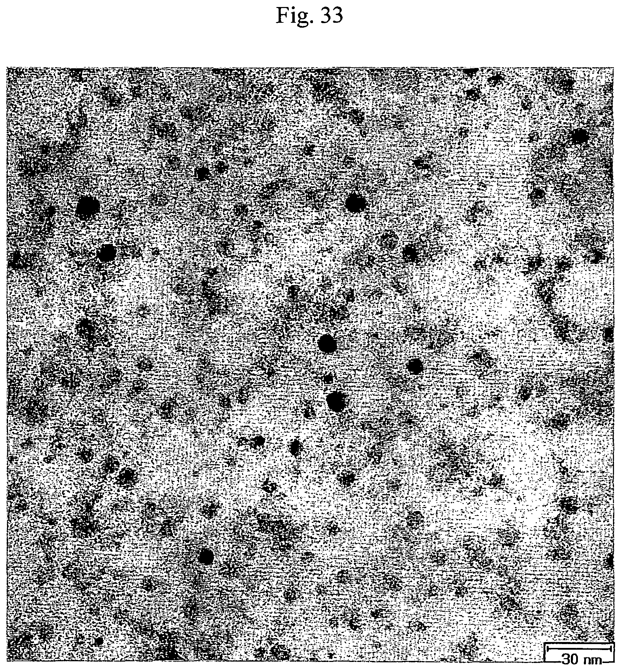

0.15 g of graft copolymer obtained in Example 12 was put in 20 ml vial and thoroughly dissolved with 3 ml of DMF (99%). 1 ml of FeCl₃ solution (0.146 g of FeCl₃/10 ml of DMF) was added thereto using a syringe and slowly stirred for about 10 minutes using a magnetic bar. The color of the solution was brown. To the mixture, 1 ml of hydrazine monohydrate (N₂H₂, Wako Junyaku Co., 98%) was slowly added as a reducing agent while stirring until the color thereof does not change any more. When the color change or bubbling no longer occurs, the resulting solution was precipitated in an excess amount of methanol, filtered, washed and dried to obtain beige powder. The size of the powder was in the range of 3 to 30 nm based on a TEM analysis. Fig. 33 is a TEM photograph of the nano-particles of the mPEG-g-MASX-iron oxide obtained above.

### <Example 21>

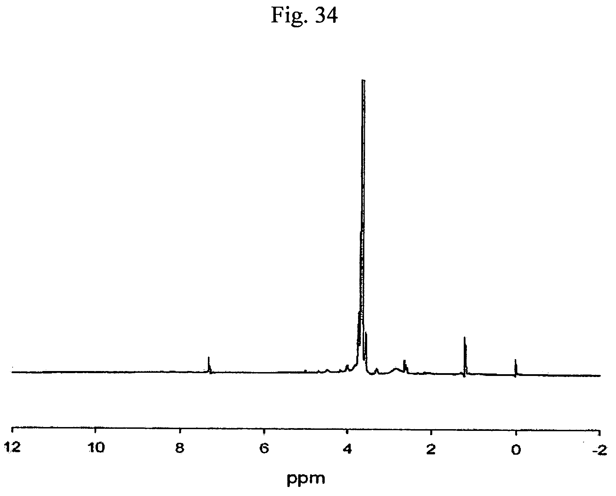

0.01 mol of mPEG-TMA (Mn= 5,200 g/mol) obtained in Example 6 and sulfadiazine (0.03 mol)/ethanol (50 ml) were put in 250 ml reactor and 100 ml of ethanol was added thereto. The mixture was refiuxed, for 12 hrs at 70 "C, precipitated in diethyl ether at room temperature and recrystallized from ethanol to obtain mPEG-sulfonamide as a solid. The number-average molecular weight of the obtained polymer was 6,000 g/mol based on a GPC analysis and the reaction yield was over 85 mol% based on the amount of mPEG used. Fig. 34 is a NMR data of the mPEG-sulfadiazine obtained above.

### <Example 22>

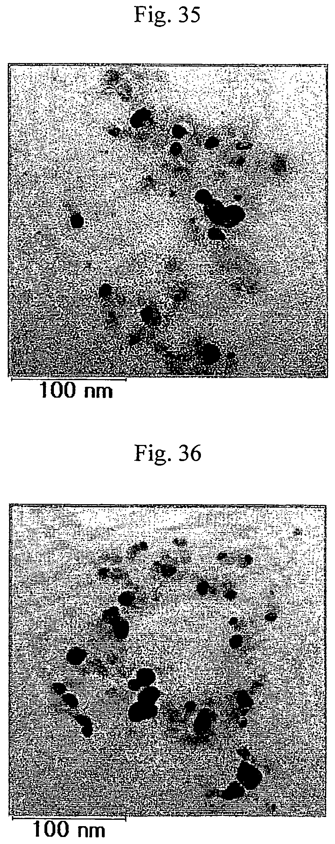

0.51 g of chain-end sulfonated mPEG (Mn= 5,100 g/mol) obtained in Example 4 was put in 20 ml vial and thoroughly dissolved with 5 ml of toluene/methanol (90/10, v/v). 0.147 g of cadmium acetate hydrate (Cd(OAc)₂- xH₂O, 6.38X10⁻⁴ mol) dissolved in 10 ml of toluene/methanol (90/10, v/v) was added thereto. To the mixture, gaseous hydrogen sulfide (H₂S) was slowly added using a syringe while stirring until the color thereof changes to yellow. It was then stirred for 6 hours. The resulting mixture was precipitated in diethyl ether to obtain yellow powder. The size of the powder was in the range of 2 to 30 nm based on a TEM analysis. Fig. 35 is a TEM photograph of the nano-particles of the ω-sulfanated mPEG-cadmium sulfide.

### <Example 23>

The same process as described in Example 22 was repeated except that 0.51 g of mPEG having a chain-end thiol group (Mn= 5,100 g/mol) obtained in Example 5 was put in 20 ml vial and 5 ml of toluene/methanol (90/10, v/v) was added thereto and thoroughly dissolved to obtain CdS powder. The size of the powder was in the range of 2 to 30 nm based on a SEM analysis. Fig. 36 is a TEM photograph of the nano-particles of the ω-thiolated mPEG cadmium sulfide obtained above.

### <Example 24>

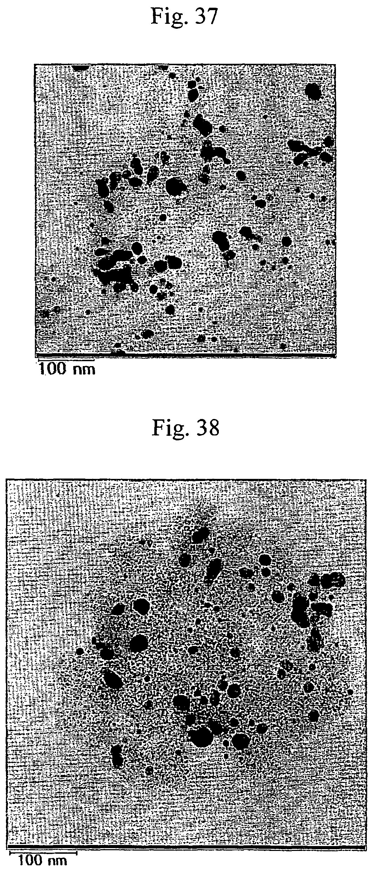

0.5 g of mPEG-TMA-vancomycin (Mn= 6,500 g/mol) obtained in Example 10 was thoroughly dissolved in 100 ml of distilled water. Then, AgNO₃ (1/5 eq. mPEG mole) and NaBH₄ (1eq. AgNO₃ mole) as a reducing agent were added thereto. The mixture was stirred at room temperature for 8 hrs. The resulting mixture was dissolved in methanol and precipitated in dimethyl ether to obtain gray powder. After dissolving the powder in methanol, the size of the powder was in the range of 5 to 15 nm based on a TEM analysis. Fig. 37 is a TEM photograph of the nano-particles of the mPEG-TMA-vancomycin obtained above.

### <Example 25>

1.5 g of mPEG-TMA-FA (Mn= 5,600 g/mol) obtained in Example 9 was dissolved in 50 ml of deoxygenated distilled water, AgNO₃ (0.01 mol) was added thereto and warmed to 40 °C while stirring. 1.5 ml of NH₄OH solution was added to the mixture, stirred for 30 minutes, cooled to room temperature and allowed to react for 24 hrs while stirring. From the resulting solution, the dark brown insoluble portion was removed by filtration and then water was removed. The resulting residue was dissolved in methanol and precipitated in dimethyl ether to obtain yellow powder. The size of the powder was in the range of 2 to 50 nm based on a TEM analysis. Fig. 38 is a TEM photograph of the nano-particles of the mPEG-TMA-folic acid-silver obtained above.

### <Example 26>

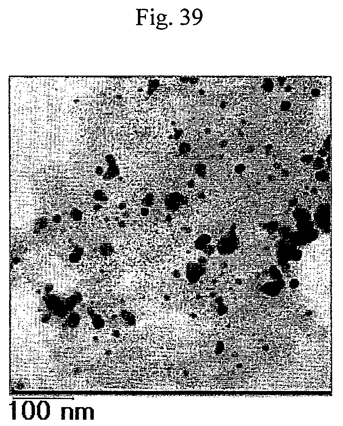

2.5 g of ω-thiolated-mPEG (Mn= 5,100 g/mol) obtained in Example 5 was dissolved in 50 ml of deoxygenated distilled water, AgNO₃ (0.01 mol) was added thereto and warmed to 40 °C while stirring. To the mixture, 1.5 ml of NH₄OH solution was added, followed by stirring for 30 minutes. The resulting mixture was cooled to room temperature and further stirred for 24 hrs. From the resulting solution, the dark brown insoluble portion was removed by filtration and then water was removed. The resulting residue was dissolved in methanol and precipitated in dimethyl ether to obtain yellow powder. The size of the powder was in the range of 2 to 50 nm based on a TEM analysis. Fig. 39 is a TEM photograph of the nano-particles of the ω-thiolated-mPEG-silver obtained above.

## Claims

1. A chain-end functionalized methoxy poly(ethylene glycol) (mPEG) of the following chemical formula 3: 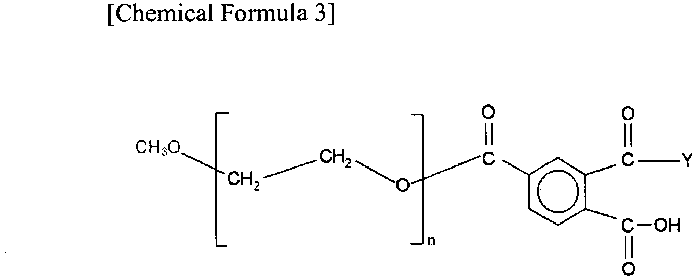 wherein,
Y is sulfonamide group such as sulfabenzene, sulfisoxazole, sulfacetamide, sulfamethizole, sulfadimethoxine, sulfadiazine, sulfamethoxy pyridazine, sulfamethazine, sulfisoimidine and sulfapyridine; a vitamin such as folic acid; or an amide or sulfonamide group-containing drug such as indisulam, doxorubicin, paclitaxel, vancomycin and amprenavir;
n is an integer in the range of 10 to 500.

2. A method for preparing a chain-end functionalized methoxy poly(ethylene glycol) (mPEG) having the structure of the following chemical formula 3: 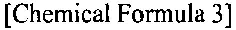 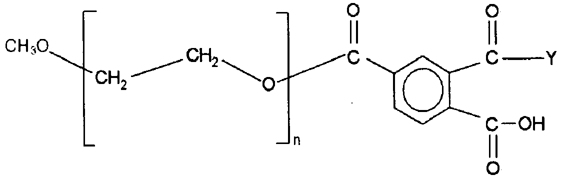 wherein,
Y is sulfonamide group such as sulfabenzene, sulfisoxazole, sulfacetamide, sulfamethizole, sulfadimethoxine, sulfadiazine, sulfamethoxy pyridazine, sulfamethazine, sulfisoimidine and sulfapyridine; a vitamin such as folic acid; or an amide or sulfonamide group-containing drug such as indisulam, doxorubicin, paclitaxel, vancomycin and amprenavir;
n is an integer in the range of 10 to 500,
comprising
(a-3) conducting reaction of a mPEG having a number-average molecular weight (Mn) of 500 to 20,000 g/mol with an alkyl alkali metal to provide a living mPEG having an end group substituted with an alkali metal cation;
(b-3) conducting reaction of the living mPEG obtained in step (a-3) with trimellitic anhydride chloride under vacuum, or argon gas stream or nitrogen gas stream; and
(c-3) conducting reaction of ω-anhydride mPEG obtained in step (b-3) with functionalization material under vacuum, or argon gas stream or nitrogen gas stream.

3. The method according to Claim 2, wherein the alkyl alkali metal of the (a-3) is one or more selected from the group consisting of alkyl lithium, diisopropylamino lithium, or an alkyl alkali metal having sodium, potassium, cesium or rubidium substituted for the lithium in the foregoing alkyl alkali metals.

4. A polymer-drug complex,
consisting of a polymer and a drug combined with the polymer, and
being the chain-end functionalized mPEG according to Claim 1 wherein Y is selected from the group consisting of a vitamin such as folic acid; and an amide group or sulfonamide group-containing drug such as indisulam, doxorubicin, paclitaxel vancomycin and amprenavir.

5. A nano-sized transition metal or metal salt particle, wherein the transition metals or metal salts are encapsulated in a micelle structure formed by the chain end-functionalized mPEG according to Claim 1 or the polymer-drug complex according to Claim 4.

6. The nano-sized transition metal or metal salt particle according to Claim 5, wherein:
(1) the transition metals or the metal salts are selected from the group consisting of Au, Ag, Pt(II), Pd(II), CdS, PbS, TiO₂, -Fe₂O₃ and Fe₃O₄.; or
(2) the particle has a size of 1 to 500 nm.

## Patentansprüche

1. Am Kettenende funktionalisiertes Methoxy-Poly(ethylenglycol) (mPEG) der folgenden chemischen Formel 3: 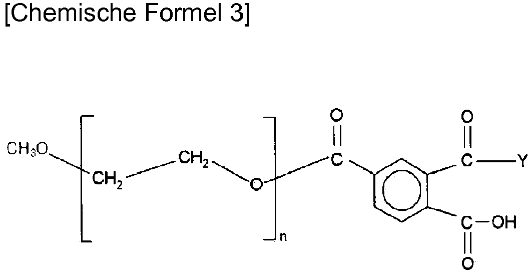 wobei
Y eine Sulfonamidgruppe wie beispielsweise Sulfabenzen, Sulfisoxazol, Sulfacetamid, Sulfamethizol, Sulfadimethoxin, Sulfadiazin, Sulfamethoxypyridazin, Sulfamethazin, Sulfisoimidin und Sulfapyridin; ein Vitamin wie beispielsweise Folsäure; oder ein Arzneimittel, das eine Amid- oder Sulfonamidgruppe enthält, wie beispielsweise Indisulam, Doxorubicin, Paclitaxel, Vancomycin und Amprenavir ist;
n eine ganze Zahl im Bereich von 10 bis 500 ist.

2. Verfahren zum Herstellen eines am Kettenende funktionalisierten Methoxy-Poly(ethylenglycol)s (mPEG), das die Struktur der folgenden chemischen Formel 3 hat: 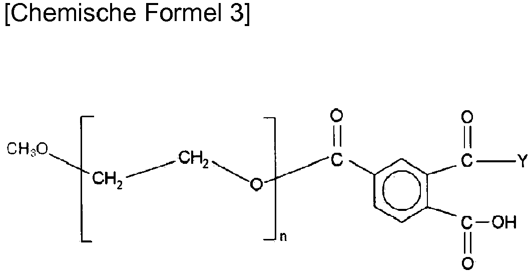 wobei
Y eine Sulfonamidgruppe wie beispielsweise Sulfabenzen, Sulfisoxazol, Sulfacetamid, Sulfamethizol, Sulfadimethoxin, Sulfadiazin, Sulfamethoxypyridazin, Sulfamethazin, Sulfisoimidin und Sulfapyridin; ein Vitamin wie beispielsweise Folsäure; oder ein Arzneimittel, das eine Amid- oder Sulfonamidgruppe enthält, wie beispielsweise Indisulam, Doxorubicin, Paclitaxel, Vancomycin und Amprenavir ist;
n eine ganze Zahl im Bereich von 10 bis 500 ist,
umfassend
(a-3) Durchführen einer Reaktion eines mPEG, das ein Zahlenmittel des Molekulargewichts (Mn) von 500 bis 20.000 g/mol hat, mit einem Alkyl-akalimetall, um ein geladenes mPEG, das eine mit einem Alkalimetallkation substituierte Endgruppe hat, bereitzustellen;
(b-3) Durchführen einer Reaktion des geladenen mPEGs, das in Schritt (a-3) erhalten wurde, mit Trimellit-anhydridchlorid unter Vakuum, oder Argongasstrom oder Stickstoffgasstrom; und
(c-3) Durchführen einer Reaktion des ω-Anhydrid-mPEGs, das in Schritt (b-3) erhalten wurde, mit Funktionalisierungsmaterial unter Vakuum, oder Argongasstrom oder Stickstoffgasstrom.

3. Verfahren gemäß Anspruch 2, wobei das Alkyl-akalimetall von (a-3) eines oder mehrere ausgewählt aus der Gruppe bestehend aus Alkyllithium, Diisopropylaminolithium, oder ein Alkyl-akalimetall, das in den vorstehenden Alkyl-akalimetallen Natrium, Kalium, Caesium oder Rubidium für das Lithium substituiert hat, ist.

4. Polymer-Arzneimittel-Komplex,
bestehend aus einem Polymer und einem Arzneimittel, das mit dem Polymer kombiniert ist und das das am Kettenende funktionalisierte mPEG gemäß Anspruch 1 ist, wobei Y aus der Gruppe bestehend aus einem Vitamin wie beispielsweise Folsäure; und einem Arzneimittel, das eine Amid- oder Sulfonamidgruppe enthält, wie beispielsweise Indisulam, Doxorubicin, Paclitaxel, Vancomycin und Amprenavir ausgewählt ist.

5. Übergangsmetall- oder Metallsalzpartikel in Nanogröße, wobei die Übergangsmetalle oder Metallsalze in einer Micellenstruktur, die durch das am Kettenende funktionalisierte mPEG gemäß Anspruch 1 oder den Polymer-Arzneimittel-Komplex gemäß Anspruch 4 geformt wird, eingekapselt sind.

6. Übergangsmetall- oder Metallsalzpartikel in Nanogröße gemäß Anspruch 5, wobei:
(1) die Übergangsmetalle oder Metallsalze aus der Gruppe bestehend aus Au, Ag, Pt(II), Pd(II), CdS, PbS, TiO₂, -Fe₂O₃ und Fe₃O₄ ausgewählt sind; oder
(2) der Partikel eine Größe von 1 bis 500 nm hat.

## Revendications

1. Méthoxy polyéthylène glycol fonctionnalisé d'extrémité de chaîne (mPEG) de la formule chimique 3 suivante : 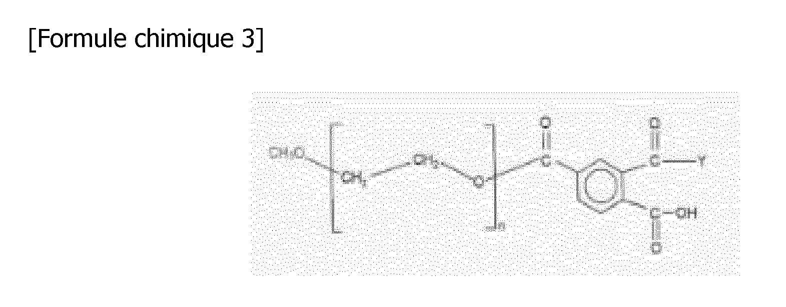 dans laquelle,
Y représente un groupement sulfonamide, tel que sulfobenzène, sulfisoxazole, sulfacétamide, sulfaméthizole, sulfadiméthoxine, sulfadiazine, sulfaméthoxy-pyridazine, sulfaméthazine, sulfisoimidine et sulfapyridine, une vitamine telle que l'acide folique ou un médicament contenant un groupement amide ou sulfonamide, tel qu'indisulam, doxorubicine, paclitaxel, vancomycine et amprénavir,
n est un nombre entier compris dans la plage de 10 à 500.

2. Procédé de préparation d'un méthoxy polyéthylène glycol fonctionnalisé d'extrémité de chaîne (mPEG) ayant la structure de la formule chimique suivante 3 : 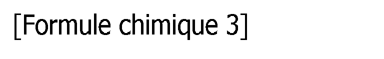 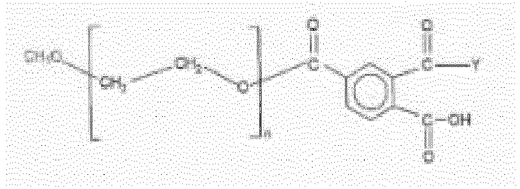 dans laquelle,
Y représente un groupement sulfonamide, tel que sulfabenzène, sulfisoxazole, suffacétamide, sulfaméthizole, sulfadiméthoxine, sulfadiazine, sulfaméthoxy-pyridazine, sulfaméthazine, sulfisoimidine et sulfapyridine, une vitamine telle que l'acide folique ou un médicament contenant un groupement sulfonamide, tel qu'indisulam, doxorubicine, paclitaxel, vancomycine et amprénavir,
n représente un nombre entier compris dans la plage de 10 à 500,
le procédé comprenant
(a-3) la réalisation de la réaction d'un mPEG ayant une masse moléculaire moyenne en nombre (Mn) de 500 à 20.000 g/mol avec un métal alkyle alcalin pour produire un mPEG vivant ayant un groupement d'extrémité substitué par un cation de métal alcalin,
(b-3) réalisation de la réaction du mPEG vivant obtenu à l'étape (a-3) avec de la chlorure d'anhydre trimellitique sous vide ou un courant gazeux d'argon ou un courant gazeux d'azote.
(c-3) réalisation de la réaction de ω-anhydride mPEG.* obtenu à l'étape (b-3) avec un matériau de fonctionnalisation sous vide ou un courant gazeux d'argon ou un courant gazeux d'azote.

3. Procédé suivant la revendication 2, dans lequel le métal alkyle alcalin de l'étape (a-3) est un ou plusieurs sélectionnés parmi le groupe constitué d'alkyl-lithium, de lithium diisopropylamine ou d'un métal alkyle alcalin ayant du sodium, du potassium, du césium ou du rubidium substitués au lithium dans les métaux alkyle alcalins ci-avant.

4. Complexe médicament/polymère,
consistant en un polymère et un médicament combiné au polymère et étant le mPEG fonctionnalisé d'extrémité de chaîne suivant la revendication 1, dans lequel Y est sélectionné parmi le groupe constitué par une vitamine, telle que l'acide folique, et un groupement amide ou un médicament contenant un groupement sulfonamide, tel qu'indisulam, doxorubicine, paclitaxel vancomoycine et amprénavir.

5. Particule de métal de transition ou de sel de métal de dimension nanométrique, dans laquelle les métaux de transition ou les sels de métaux sont encapsulés dans une structure micellaire formée par le mPEG fonctionnalisé d'extrémité de chaîne suivant la revendication 1 ou dans le complexe médicament/polymère suivant la revendication 4.

6. Particule de métal de transition ou de sel de métal de dimension nanométrique suivant la revendication 5, dans laquelle :
(1) les métaux de transition ou les sels de métaux sont sélectionnés parmi le groupe constitué par Au, Ag, Pt(II), Pd(II), CdS, PbS, TiO₂, -Fe₂O₃ et FE₃O₄, ou
(2) la particule présente une dimension de 1 à 500 nm.
